Europäisches Patentamt

European Patent Office

Office européen des brevets

(19)

(11) Numéro de publication: **0 044 111**

**A1**

# (12) DEMANDE DE BREVET EUROPEEN

(21) Numéro de dépôt: **81200765.6**

(51) Int. Cl.³: **C 23 G 5/02**

(22) Date de dépôt: **06.07.81**

(30) Priorité: **15.07.80 FR 8015767**

(43) Date de publication de la demande:
**20.01.82 Bulletin 82/3**

(84) Etats contractants désignés:
**AT BE CH DE FR GB IT LI LU NL SE**

(71) Demandeur: **SOLVAY & Cie (Société Anonyme)**
**Rue du Prince Albert, 33**
**B-1050 Bruxelles(BE)**

(72) Inventeur: **Walraevens, René**
**Avenue des Neuf Provinces 3**
**B-1080 Bruxelles(BE)**

(72) Inventeur: **Servais, Michel**
**Chaussée de Malines 315**
**B-1950 Kraainem(BE)**

(74) Mandataire: **Eischen, Roland**
**Solvay & Cie Département de la Propriété Industrielle**
**Rue de Ransbeek 310**
**B-1120 Bruxelles(BE)**

(54) Compositions stabilisées de 1,1,1-trichloroéthane.

(57) L'invention concerne des compositions de 1,1,1-trichloroéthane stabilisées contenant un nitroalcane et du 2-méthyl-2,3-époxybutane.

Ces compositions conviennent bien pour dégraisser des pièces métalliques mouillées d'huiles de coupe.

EP 0 044 111 A1

Compositions stabilisées de 1,1,1-trichloroéthane

Cas S.80/7

SOLVAY & Cie (Société Anonyme)

La présente invention concerne des compositions stabilisées de 1,1,1-trichloroéthane, utilisables pour le dégraissage des métaux en conservant un pH constant.

On sait que le 1,1,1-trichloroéthane, qui est utilisé notamment pour le dégraissage des métaux, le nettoyage à sec des textiles, et dans les aérosols, pose un problème de stabilité et de corrosion tout particulier. L'emploi, pour stabiliser le 1,1,1-trichloroéthane, des stabilisants habituellement utilisés pour d'autres hydrocarbures chlorés ne donne pas, en général, des résultats satisfaisants. Ce phénomène a été attribué au fait qu'il existe une grande réactivité entre les métaux et le 1,1,1-trichloroéthane, réactivité qui donne lieu dans un délai très court à la décomposition du 1,1,1-trichloroéthane et à l'attaque du métal.

On a déjà proposé, pour stabiliser le 1,1,1-trichloroéthane, d'utiliser conjointement des composés époxydés et des nitroalcanes (brevet des Etats-Unis US-A-3 049 571 déposé le 18 mars 1960 et cédé à DOW CHEMICAL CO; brevets des Etats-Unis US-A-3 532 761 et US-A-4 026 956 et brevet belge BE-A-763 703 déposés respectivement les 7 mai 1969, 11 juin 1975 et le 3 mars 1970 dont le titulaire est PITTSBURGH PLATE GLASS CO). Selon ces brevets, différents composés époxydés peuvent être mis en oeuvre à cet effet. Cependant, comme seul l'époxybutane est disponible à des conditions commercialement avantageuses, c'est toujours ce composé qui est utilisé en pratique.

Si la stabilité de pH des compositions de 1,1,1-trichloroéthane stabilisées au moyen d'époxybutane peut être considérée dans la plupart des cas comme satisfaisante, il subsiste cependant des problèmes de stabilité lorsque ces compositions sont utilisées pour dégraisser des pièces métalliques mouillées par des

0044111

- 2 -

huiles de coupe. En effet, dans ce cas, on observe, suivant la nature de l'huile de coupe utilisée, que la composition de 1,1,1-trichloroéthane devient acide ou basique, ce qui nécessite une adaptation de la formule de stabilisation cas par cas.

La présente invention vise à fournir des compositions de 1,1,1-trichloroéthane dont le pH ne varie pas, même lorsqu'elles sont utilisées pour dégraisser des pièces métalliques mouillées d'huiles de coupe de natures diverses.

Elle concerne à cet effet des compositions de 1,1,1-trichloroéthane contenant au moins un nitroalcane et au moins un composé époxydé qui est le 2-méthyl-2,3-époxybutane.

Le nitroalcane des compositions selon l'invention contient habituellement de 1 à 4 atomes de carbone. De préférence, c'est le nitrométhane, le nitroéthane, le nitropropane 1 ou 2 ou un mélange de ces composés. Tout particulièrement préférés sont le nitrométhane et le nitroéthane.

La quantité de nitroalcane varie habituellement entre 0,001 mole et 1 mole par litre de 1,1,1-trichloroéthane. De préférence, cette quantité est située entre 0,01 et 0,5 mole par litre. Tout particulièrement préférées sont des quantités comprises entre 0,05 et 0,3 mole par litre de 1,1,1-trichloroéthane.

Les quantités de 2-méthyl-2,3-époxybutane, également appelé oxyde d'isoamylène, sont les mêmes que celles définies ci-dessus pour les nitroalcanes.

Outre les composés précités, les compositions peuvent contenir un ou plusieurs autres stabilisants habituels du 1,1,1-trichloroéthane tels que des alcools, des esters, des nitriles, des éthers internes et des cétones ainsi que des composés époxydés tels que l'oxyde d'isobutène. On préfère toutefois les compositions contenant le 2-méthyl-2,3-époxybutane comme seul composé époxydé.

Les alcools peuvent être saturés ou insaturés. Les alcools préférés contiennent de 1 à 7 atomes de carbone et peuvent être substitués ou non. Généralement, ce sont des alcools non substitués.

Des exemples d'alcools saturés sont le méthanol, l'éthanol, le propanol, l'isopropanol, le butanol 1, le butanol 2, l'alcool butylique tertiaire, les pentanols tels que l'alcool amylique tertiaire, l'éther monométhylique, monoéthylique, monopropylique ou monobutylique de l'éthylène glycol, la glycérine, l'éthylène-glycol, le propylèneglycol, la 2-hydroxy-2-méthyl-3-butanone, le 3,3-diméthoxy-2-méthylbutanol-2 et le 3-amino-2-méthylbutanol-2. D'excellents résultats ont été obtenus avec les propanols, les butanols tertiaire et secondaire et le pentanol tertiaire. L'alcool saturé préféré est le butanol tertiaire.

Les alcools insaturés habituellement utilisés sont des alcools non substitués contenant de 3 à 6 atomes de carbone. Parmi ceux-ci on préfère l'alcool allylique, le 2-méthyl-3-butène-2-ol. et le 2-méthyl-3-butyne-2-ol. L'alcool insaturé préféré est le 2-méthyl-3-butyne-2-ol.

Comme ester, on utilise le plus souvent des mono- et des di-esters contenant de 2 à 8 atomes de carbone saturés ou non, pouvant être substitués par des halogènes ou des groupes hydroxyles tels que les formiates d'éthyle, de propyle, d'isopropyle, de butyle, de sec-butyle, de tert-butyle, d'amyle, d'hexyle et de cyclohexyle, les acétates de méthyle, d'éthyle, de propyle, d'isopropyle, de butyle, d'amyle, d'éthynyle et de propynyle, les propionates de méthyle, d'éthyle, de propyle, d'isopropyle, de butyle, d'amyle et d'éthynyle, les butyrates de méthyle, d'éthyle, de propyle, d'isopropyle et de 2-propynyle, les acrylates et méthacrylates de méthyle et d'éthyle, de propyle, d'isopropyle, de butyle, de sec-butyle et de tert-butyle, les crotonates et isocrotonates de méthyle et d'éthyle, les sorbinates d'éthyle et de méthyle, le diformiate d'éthyle et le diacétate d'éthyle. Les mono-esters non substitués contenant de 4 à 6 atomes de carbone conviennent bien. D'excellents résultats ont été obtenus avec l'acétate d'éthyle, l'acétate d'isobutyle, l'acétate de n-propyle, le méthacrylate de méthyle et l'acétate de n-butyle. Le méthacrylate de méthyle, l'acétate d'éthyle et l'acétate de n-propyle se sont révélés particulièrement adéquats.

Les nitriles le plus souvent mis en oeuvre sont des composés aliphatiques ou aromatiques, saturés ou non saturés, éventuellement substitués, contenant de 2 à 7 atomes de carbone tels que l'acétonitrile, le propionitrile, l'acrylonitrile, le butyronitrile, le méthacrylonitrile, le malononitrile, le benzonitrile, le diméthylaminoacétonitrile, le méthylaminopropionitrile, le diméthylaminopropionitrile, le diéthylaminoacétonitrile, le méthyléthylaminoacétonitrile, le thiodipropionitrile, le tétracyanoéthylène, le tétracyanoquinodiméthane, le tétracyanodithiine, le 1,2-dicyano-1,2-bis(trifluorométhyl)éthylène, le cyanobenzaldéhyde, le nitrobenzonitrile, la cyanopyridine. Parmi ceux-ci l'acétonitrile, le propionitrile et l'acrylonitrile conviennent le mieux.

Les éthers internes particulièrement adéquats comprennent les éthers mono- ou polycycliques, pouvant contenir éventuellement d'autres hétéroatomes, contenant un ou des cycles à 5 ou 6 atomes de carbone saturés ou non, substitués ou non tels que le 1,3-dioxanne, le 1,4-dioxanne, le 1,3,5-trioxanne, le 1,3-dioxolanne, le dioxène, le dioxadiène, le thioxanne, l'oxazole, le tétrahydrofuranne, l'oxyde de diphényle, etc.

Comme cétones, on peut utiliser les cétones contenant de 3 à 7 atomes de carbone substituées ou non, telles que par exemple l'acétone, la méthyléthylcétone, la diéthylcétone, la méthylpropylcétone, la méthylisopropylcétone, la 1(2 furyl)propanone, la 3-hydroxy-2,5-hexanedione, la 2-méthyl-2-hydroxy-4-pentanone, etc.

D'autres composés et notamment les alcoxyalcanes tels que les alkoxyéthanes, les nitrates tels que les nitrates d'alkyle contenant de 1 à 5 atomes de carbone, les amines tels que les amines aliphatiques contenant de 2 à 6 atomes de carbone, les pyrroles tels que le N-méthylpyrrole et d'autres composés aromatiques tels que le toluène ou aliphatiques tels que le diisobutylène peuvent être également incorporés dans la formule de stabilisation du 1,1,1-trichloroéthane mise finalement en oeuvre.

Les différents autres stabilisants utilisés sont généralement présents dans des quantités comprises entre 0,01 et 2 mole par litre de 1,1,1-trichloroéthane et de préférence dans des quantités comprises entre 0,1 et 0,8 mole par litre.

Des compositions très efficacement stabilisées selon l'invention contiennent outre le 2-méthyl-2,3-époxybutane et du nitrométhane ou nitroéthane, un alcool saturé, et plus particulièrement l'alcool butylique tertiaire, et un alcool insaturé, et plus particulièrement le 2-méthyl-3-butyne-2-ol.

Les compositions de 1,1,1-trichloroéthane stabilisées selon l'invention peuvent être utilisées dans toutes les applications requérant une bonne stabilisation du 1,1,1-trichloroéthane mis en oeuvre. Elles sont de préférence utilisées pour dégraisser les métaux et, plus particulièrement, pour dégraisser des métaux mouillés par des huiles de coupe. Par huiles de coupe, on entend désigner tout type d'huile ou de graisse utilisé lors d'opérations telles que l'usinage, l'alèsage, le polissage, l'étirage et le tréfilage de métaux.

Les exemples qui suivent n'ont aucun caractère limitatif.

Exemple 1

Cet exemple est fourni à titre comparatif.

Dans un ballon de 500 cm$^3$, équipé d'un thermomètre et muni d'un appareil d'extraction du type SOXHLET d'une capacité de 100 cm$^3$ lui-même surmonté d'un réfrigérant, maintenus à l'abri de la lumière, on introduit 300 cm$^3$ de trichloroéthane stabilisé avec 0,541 mole/l d'alcool butylique tertiaire, 0,164 mole/l de nitrométhane, 0,208 mole/l de 2-méthyl-3-butyne-2-ol. et 0,079 mole/l d'époxybutane, et 75 cm$^3$ d'une huile de coupe (respectivement de l'huile de caléol pour l'essai 1, du stéarate de calcium pour l'essai 2 et du polybutylcuprisil pour l'essai 3).

Le mélange est porté à l'ébullition pendant 72 heures et le chauffage est réglé de façon à réaliser avec des intervalles de 30 minutes le remplissage et une vidange complète de l'extracteur. Par conséquent, la concentration en huile de coupe dans le mélange varie en continu entre 20 et 27,6 % en volume imitant ainsi les

- 6 -

conditions existantes dans les machines industrielles de dégraissage en phase vapeur.

Au terme des 72 heures d'ébullition, le contenu de l'extracteur est vidé dans le ballon, le réfrigérant est monté en oblique et l'on récupère le solvant par distillation. La dégradation de , ce solvant est déterminée par mesure de l'acidité et les valeurs sont rassemblées au Tableau I ci-dessous.

TABLEAU I

|  | Essai 1 | Essai 2 | Essai 3 |
|---|---|---|---|
| acidité en mg/1 | 59 | −14* | 0 |

* la solution est devenue alcaline.

Exemple 2

On répète les opérations de l'exemple 1 mais en opérant avec du 1,1,1-trichloroéthane stabilisé avec 0,541 mole/1 d'alcool butylique tertiaire, 0,164 mole/1 de nitrométhane, 0,208 mole/1 de 2-méthyl-3-butyne-2-ol. et 0,079 mole/1 de 2-méthyl-2,3-époxybutane.

La dégradation de ce solvant est déterminée par mesure de l'acidité et les valeurs sont rassemblées au Tableau II ci-après.

TABLEAU II

|  | Essai 1 | Essai 2 | Essai 3 |
|---|---|---|---|
| acidité en mg/1 | 0 | 0 | 0 |

La comparaison des résultats des exemples 1 et 2 montre qu'une composition de 1,1,1-trichloroéthane stabilisée par du nitrométhane et du 1,2-époxybutane peut être attaquée par l'action de diverses huiles de coupe et ne convient que pour certaines huiles de coupe non agressives alors que la composition selon l'invention dans laquelle le 1,2-époxybutane est remplacé par une quantité équivalente de 2-méthyl-2,3-époxybutane convient dans tous les cas.

REVENDICATIONS

1 - Compositions stabilisées de 1,1,1-trichloroéthane contenant au moins un nitroalcane et au moins un composé époxydé caractérisées en ce que le composé époxydé est le 2-méthyl-2,3-époxybutane.

2 - Compositions selon la revendication 1, caractérisées en ce que le nitroalcane est le nitrométhane ou le nitroéthane.

3 - Compositions selon les revendications 1 ou 2, caractérisées en ce que le nitroalcane et le 2-méthyl-2,3-époxybutane sont présents dans des quantités comprises entre 0,001 et 1 mole par litre de 1,1,1-trichloroéthane.

4 - Compositions selon l'une quelconque des revendications 1 à 3, caractérisées en ce qu'elles contiennent en outre un ou plusieurs autres stabilisants choisis parmi les alcools, les esters, les nitriles, les éthers internes et les cétones.

5 - Compositions selon la revendication 4, caractérisées en ce que les alcools sont des alcools saturés ou insaturés non substitués.

6 - Compositions selon la revendication 5, caractérisées en ce que les alcools sont l'alcool butylique tertiaire ou le 2-méthyl-3-butyne-2-ol.

7 - Compositions selon la revendication 6, caractérisées en ce qu'elles contiennent de l'alcool butylique tertiaire et du 2-méthyl-3-butyne-2-ol.

8 - Compositions selon l'une quelconque des revendications 4 à 7, caractérisées en ce que les autres stabilisants sont présents à raison de 0,01 à 2 mole par litre de 1,1,1-trichloroéthane.

9 - Compositions selon l'une quelconque des revendications 1 à 8, caractérisées en ce qu'elles ne contiennent que du 2-méthyl-2,3-époxybutane comme composé époxydé.

Office européen
des brevets

**RAPPORT DE RECHERCHE EUROPEENNE**

EP 81 20 0765

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int. Cl.³) |
|---|---|---|---|
| DX | US - A - 3 049 571 (W.E. BROWN)<br>* Revendication 13; colonne 2, lignes 46-54; colonne 2, lignes 41-45 * | 1-3,9 | C 23 G  5/02 |
| X | FR - A - 1 444 410 (PPG)<br>* Exemple 2; page 2; page 3, colonne de droite, ligne 2 * | 1-9 | |
| D | FR - A - 2 042 444 (PPG)<br>* Page 5, ligne  25  à page 6, ligne 24 *<br>& US - A - 3 532 761 | 1-9 | **DOMAINES TECHNIQUES RECHERCHES (Int. Cl.³)**<br><br>C 23 G  5/02<br>C 07 C 19/02<br>C 07 C 17/42 |
| | US - A - 3 767 585 (S. SAWABE)<br>* Revendication 11; exemples 10,11,12, page 3 * | 1 | |
| D | US - A - 4 026 956 (PPG)<br>*Revendications 6 et 13 * | 1 | |
| A | FR - A - 2 429 767 (RHONE POULENC)<br>---- | | **CATEGORIE DES DOCUMENTS CITES**<br><br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons<br><br>&: membre de la même famille, document correspondant |

Le présent rapport de recherche a été établi pour toutes les revendications

| Lieu de la recherche | Date d'achèvement de la recherche | Examinateur |
|---|---|---|
| La Haye | 13-10-1981 | TORFS |

OEB Form 1503.1  06.78